# EUROPEAN PATENT SPECIFICATION

(11) **EP 1 522 267 B1**
(45) Date of publication and mention of the grant of the patent: **14.01.2009**
(21) Application number: 04405626.5
(22) Date of filing: 06.10.2004
(51) Int. Cl.: A61B 17/64, A61B 17/86

(54) **External fixation elements**
Äusserliche Befestigungsvorrichtung
Elements de fixation externe

(30) Priority: 06.10.2003 EP 03405713
(43) Date of publication of application: 13.04.2005
(73) Proprietor: Stryker Trauma SA, 2545 Selzach (CH)
(72) Inventor: Burgherr, Vinzenz, 3005 Bern (CH); Lutz, Christian, 4500 Solothurn (CH); Palefroy, Olivier, 74420 Villard (FR)
(74) Representative: Liebetanz, Michael

(56) References cited:
- EP-A- 0 717 968
- CH-A- 303 453
- FR-A- 2 338 692
- US-A- 4 258 708
- US-A- 4 757 809
- US-A- 6 045 553
- US-A1- 2003 149 429

## Description

The invention relates to a clamp for an external fixation system according to the preamble of claim 1.

Magnetic resonance imaging (MRI) is a well known diagnostic tool in the field of medicine. It is equally known to surgeons that it may be dangerous for patients wearing metallic devices inside or outside their body to be examined with a MRI device (inside or outside the coil).

It is known in the prior art, e.g. from FR 2,703,580, to use antimagnetic implants, e.g. made in polyethylene, and to use additional radio-opaque markers to get an image of the implant. This allows to declare such an implant MRI-safe.

US 2002/042619 discloses a headframe for use with a stereotactic system. It is mentioned in the last paragraph of the description of said document that the frame body and swivel arms need not to be made from aluminum. Instead, the frame body and swivel arms as well as other components of the headframe may be formed of a non-magnetic material such as plastic, ceramic, or other composite such that the headframe is compatible with MRI, CT, X-ray and magnetic stereotactic devices/procedures.

Document CH 303453 discloses an external fixation system comprising a rod and two clamps, the clamps comprising on the inner side of the jaws an insulating material.

Document EP 0 7171 968 discloses an external fixation system comprising a rod and clamps, the rod captured by and extending between the clamps and being made from a non-ferromagnetic material core surrounded by a non-electrically conductive sheath.

In the field of elements constituting an external fixation device or to be used with an external fixation device it is known to use e.g. rods in stainless steel. However, the use of such ferromagnetic rods does not allow MRI examinations. As alternative, rods in aluminum or in carbon fibers may be used. Rods made from the latter named materials are not ferromagnetic. However, within the field of external fixation, the combination of these elements, e.g. rods with clamps and pins, constitute in itself or together with the body of the patient a closed conductive loop.

For MRI safety it is equally important not to interfere with the static field, the gradient field as well as the RF-field, therefore such external fixation elements cannot be used in MRI environments.

In view of this prior art the object of the invention is to provide external fixation elements, wherein a patient carrying such a structure can safely be examined within a MRI device.

During MRI scanning critical heat can be generated in such external fixation frames that can cause burns, necrosis or nerve stimulation in the tissue of the human body. Furthermore a torque may be generated resulting in a vibration or movement of the device.

It is another object of the invention to provide external fixation elements with the least possible magnetic field interactions (i.e. deflection and/or torque), heating and nerve stimulation. It is a further object of the invention to limit artifacts in the generated MRI images due to the external fixation elements.

The field strength, i.e. the highest strength of the static magnetic field of a MR system, is often between 1.5 Tesla and 3.0 Tesla, more often around 2.0 Tesla. However elements according to the invention can also be used in association with very high-field-strength MR systems having a field strength of up to 8.0 Tesla.

A clamp having the features mentioned in the preamble of claim 1, using two wedge elements is known from US-A-4, 757, 809.

The above-mentioned objects are achieved for a clamp through the features of claim 1. An external fixation system using such a clamp is claimed in claim 4.

Further objects and advantages are achieved through the features of the subclaims and exemplary embodiments of the invention are disclosed in the following description in which:
- Fig. 1: is a schematic view of a rod according to the invention for use in an external fixation device,
- Fig. 2: is a view of several fixation elements according to the invention with a rod according to Fig. 1, and
- Fig. 3: is a representation of a clamp according to the invention, seen in section in the left and laterally in the right part of the drawing.

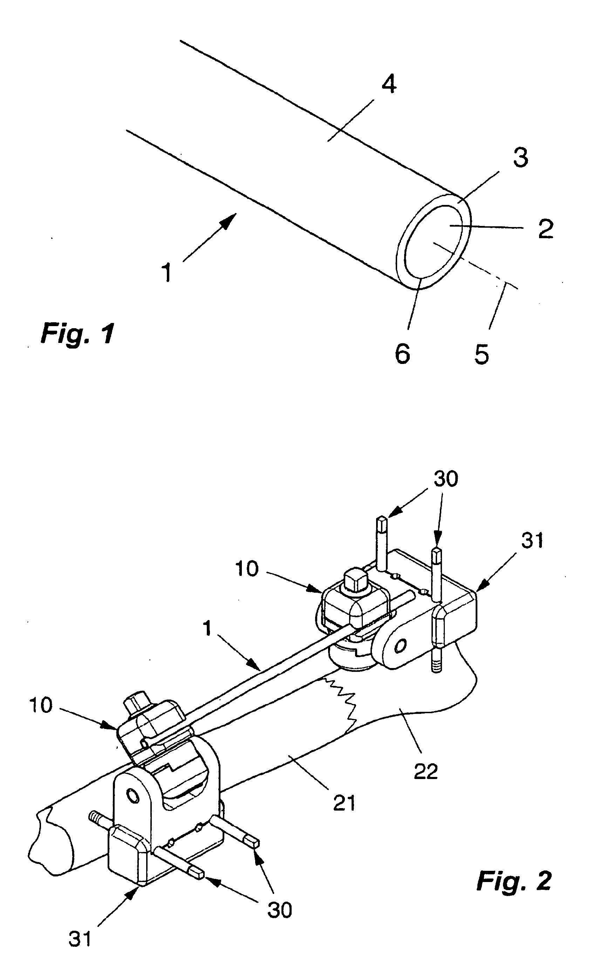

Fig. 1 shows a schematic view of a rod 1 for use in an external fixation device. The core has received the reference numeral 2. A non-conductive sheath 3 encloses the core 2. The cylindrical surface 4 of the sheath may be smooth or comprise a grid of high spots. Preferably rod 1 is available with different diameters depending on its use in an external fixation device, e.g. as thicker rods 1 and thinner pins 31 as shown in Fig. 2.

The rod 1 according to the invention is to be used with further elements of an external fixation system or frame, e.g. clamps 10 and 31 as shown in Fig. 2. Fig. 2 shows a schematic view of the use of a external fixation system with a broken bone 20 using several fixation elements according to the invention. Pins 30 are drilled into the bone parts 21 and 22, respectively and held within clamp parts 31. The clamp parts 31 are mounted together with clamps 10. The two clamps 10 are connected via the rod 1 oriented mainly in parallel to the broken limb.

The clamps 10 and 31 can be made of titanium. Such clamps 10 are then non-ferromagnetic but electrically conductive. However, the use of such conductive clamps 10 and 31 does not close the external fixation frame to a closed electrical loop, since the rods 1 are not conductive. Therefore the combination of the disclosed rods 1 from an anti-ferromagnetic material with clamps 10 and 31 and pins 30 are MRI-safe. There are almost no induced currents. The use of the non-conductive elements 1 disrupts the closed loop usually formed by rod 1, clamp parts 10 and 31, pin 30 and the human body, here represented through the bone parts 21 und 22. The use of non magnetic material for clamp 31 also disrupts the smaller loop formed by the two pins 30 together with one clamp part 31 and the bone.

Beside the use of conductive pins 30 it is in principle possible to use shielded or non conductive materials for said pins 30.

On the other side, it is also possible to provide not conductive clamps 10, e.g. titanium clamp bodies covered at least on the inside with an insulation sheath 34, i.e. around the jaws 40 of the clamp. The insulation sheath 33 is also provided on the outside of the clamp.

Both solutions (clamp and/or rod) interrupt the electric loop of the frame and body of a patient and prevent serious induced currents.

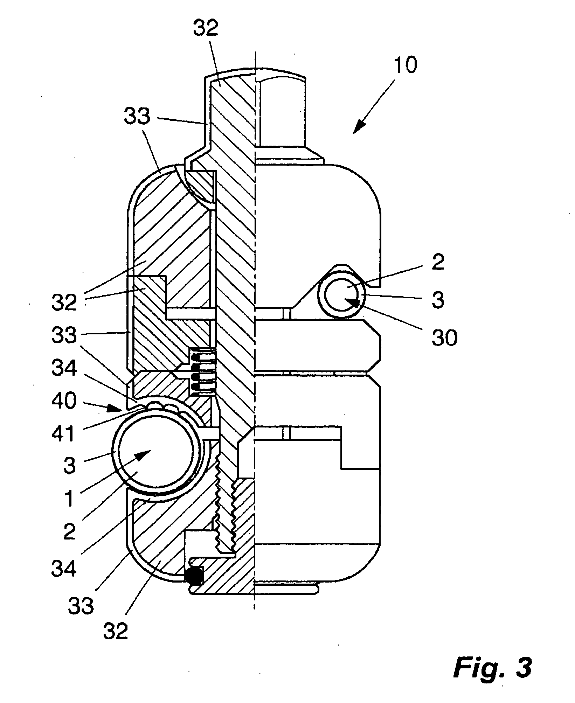

A clamp 10 with an insulation sheath 34 and 33 is shown in Fig. 3 being a representation of the clamp 10, seen in section in the left and laterally in the right part of the drawing. The elements 32 constituting the core of the clamp 10 can be in carbon fiber or titanium, although the use of aluminum is preferred.

The different constituting parts of clamp 10 are known to someone skilled in the art and are explained in US 6,080,153.

In the case that the clamps 10 have on the inner side of the jaws 40 some teeth 41 gripping the outer surface of the sheath 3 of the rod 1, the insulation 34 as shown in Fig. 3 may function in a different was as explained below. Since the rod 1 comprises the core 2 and the sheath 3, the clamp's teeth 41 enter into the sheath 3. The teeth 41 can be made of the core material of the clamp 10 for use with said rod 1 are made of titanium or aluminum and are as such not ferromagnetic, however conductive. The teeth 41 of the jaws 40 have a length of e.g. 0,3 millimeter, wherein the sheath 3 has a radial thickness of e.g. 0,7 millimeter. In this way it is ensured that the possibly conductive rod core 2 do not come in contact with the conductive teeth 41. The same apply for teeth which would be used to hold the pin 30. However in other embodiments the insulation sheath 41 may well extend also in the area of the jaws 40.

Referring to Fig. 1 possible stresses on a rod are evaluated. One of the stresses a system may receive is a torsion exerted via two clamps 10, i.e. one end of the rod 1 is rotated around his main axle 5 while the opposite end of the rod 1 is held or rotated in the opposite direction. If the torque would now be increased until the system breaks, what will not happen to a surgeon using the usual tools, there are two possible scenarios. In one case it would be the sheath 3 which separates from the core 2 of the rod 1, wherein the teeth 41 of the jaws 40 or the inner surface of the jaws 40 are holding the sheath 3 fast. The second possibility resides in the fact that the teeth 21 of the jaws 20 of the clamp 10 are gliding along the circumference of the sheath 3.

According to a preferred embodiment the rod 1 or pin 30 is designed in such a way that the clamp 10 will start to glide along the circumference of the sheath 3 before the surfaces between core 2 and sheath 3 are separating upon an growing torque. In such a way the rod 1 remains intact in the case that excessive torque stresses are exerted on said rod 1. The same apply in case of tensile forces acting along the longitudinal axle 5.

The core 2 may be formed with a protrusion process. Through resin transfer moulding (RTM) a sheath 3 having a known resistivity is molded with the core 2 allowing an interaction between a e.g. fiber carbon core 2 and the sheath 3 at the intermediate surface 6.

The core 2 may be non-metallic or metallic, conductive or non-conductive. Its main function is to provide stiffness.

The electrical resistivity of the rods 1 or pins 30 shall preferably be greater than 1 kOhm·cm.

The torsional stiffness for rods with 5 mm diameter shall preferably be greater than 0.2 Nm2. The torsional strength for such rods shall preferably be greater than 2 Nm. The bending stiffness for such rods shall preferably be greater than 2.5 Nm2. The bending strength for such rods shall preferably be greater than 15 Nm.

The torsional stiffness for rods with 8 mm diameter shall preferably be greater than 1.5 Nm2. The torsional strength for such rods shall preferably be greater than 11 Nm. The bending stiffness for such rods shall preferably be greater than 20 Nm2. The bending strength for such rods shall preferably be greater than 40 Nm.

The rods 1 are especially MRI safe for the patient when used in any frame configuration for the upper & lower extremities and pelvis, and especially when used in conjunction with Hoffmann II clamps of applicant's MRI safe product line, wherein the following MRI field parameters apply:
Static field : max. 2 Tesla
Time-varying field : max. 20 Tesla/sec.
Specific absorption rate (SAR): max. 0.4 Watts/kg averaged over the whole body.

A device is considered MRI safe if there is:
- no risk of limb movements due to forces exhibited on the materials of the frame,
- no risk of nerve or muscle stimulation due to time-varying field,
- no risk of all body temperature rise greater than 1°C, due to induced current and temperature in the frame being a consequence of the RF field.

It is important to note that any orthopedic frame for external fixation build with the elements described in this application and according to the appended claims can be considered MRI safe irrespective of the position of the frame, i.e. the device can be used for the external fixation of any broken limb of the patient.

The improvement to the MRI Safe invention relates also to the choice of material and the manner in which that material was applied to the carbon core. A higher modulus carbon fiber is used because the size of the carbon core must be reduced so that once the second material is applied to it, the resulting product has the same size as rods traditionally used by surgeons in external fixation systems. The reduced size, high modulus carbon core can be inserted into a sheath or sock of braided Vectran. Resin is "infused" through the braided Vectran material, a vacuum being used to pull the resin through the sock. There is an additional heating process step by which the resin is cured. The Vectran material, resin and carbon core are heated to about 160°C.

The MRI Safe device according to the invention is gamma resistant, nonconductive, nonmagnetic, and radiolucent. If gamma resistance is not crucial Kevlar can be used as sheath material. Kevlar sheaths have shown to discolor over time, therefore indicating the age of the product. This may be undesirable.

With respect to Vectran as choice for the material of the non-conductive sheath part, the thickness of the sheath is important because if it becomes too thick, then it becomes conductive, which is an undesirable property.

## Claims

1. A clamp (10) for an external fixation system, adapted to clamp a rod (1) or pin (30), comprising an anti-magnetic core body (32) and a non-conductive surface (33) forming a sheath part covering essentially the exterior surfaces of the core body (32), **characterized in that** the clamp (10) comprises jaws (40) having teeth (41) on the clamping surface provided to capture a rod (1) or pin (30), wherein the length of said teeth (41) is smaller than the thickness of the insulating sheath (3) of said rod (1) or pin (30).

2. The clamp (10) according to claim 1, wherein the anti-magnetic core body (32) is made of titanium or aluminum or carbon fiber and/or the non-conductive sheath part (33) is made of resin material.

3. The clamp (10) according to claim 2, wherein the anti-magnetic core part (32) is made of carbon fiber and the non-conductive sheath part (33) is made of co-extruded resin material within a resin transfer moulding (RTM) process.

4. A MRI safe external fixation system comprising:
at least two rod clamps (10) according to one of claims 1 to 3, and
at least one rod (1) or pin (30) to be captured by and extending between said rod clamps (10), said rod or pin made from a non-ferromagnetic material core (2) surrounded by a non-electrically conductive sheath (3).

5. The external fixation system according to claim 4, wherein the core (2) and sheath (3) of a rod (1) or pin (30) are holding together upon exertion of a torque around the main axle (5) of the rod (1) or pin (30) in such a way that any of said clamps (10) will start to glide along the circumference of the sheath (3) before the surfaces between core (2) and sheath (3) are separating upon an increasing torque.

6. The external fixation system according to one of claims 4 or 5, wherein the anti-magnetic core body (2, 32) of any of said clamps (10) and any rod (2) or pin (30) is made of titanium or aluminum or carbon fiber and/or the non-conductive sheath part (3, 33) is made of resin material.

7. The external fixation system according to claim 6, wherein the anti-magnetic core body (2, 32) is made of carbon fiber and the non-conductive sheath part (3, 33) is made of co-extruded resin material within a resin transfer moulding (RTM) process.

## Patentansprüche

1. Klemmelement (10) für ein äusseres Befestigungssystem, angepasst, um einen Stab (1) oder Stift (30) zu klemmen, umfassend einen antimagnetischen Kernkörper (32) und eine nicht leitende Oberfläche (33), die einen Hüllenteil ausbildet, der im Wesentlichen die äusseren Oberflächen des Kernkörpers (32) abdeckt, **dadurch gekennzeichnet, dass** das Klemmelement (10) Backen (40) umfasst, welche Zähne (41) auf der Klemmoberfläche aufweisen, die vorgesehen sind, um einen Stab (1) oder Stift (30) festzuhalten, wobei die Länge der besagten Zähne (41) kleiner ist als die Dicke der isolierenden Hülle (3) des besagten Stabes (1) oder Stiftes (30).

2. Klemmelement (10) nach Anspruch 1, bei dem der antimagnetische Kernkörper (32) aus Titan oder Aluminium oder Kohlefaser besteht und/ oder der nichtleitende Hüllenteil (33) aus einem Harzmaterial besteht.

3. Klemmelement (10) nach Anspruch 2, bei dem der antimagnetische Kernteil (32) aus Kohlefaser besteht und der nichtleitende Hüllenteil (33) aus co-extrudiertem Harzmaterial innerhalb eines Harzübertragungsgussverfahrens (RTM-Verfahren) hergestellt worden ist.

4. Ein MRI sicheres äusseres Befestigungssystem, umfassend:
- mindestens zwei Stab-Klemmelemente (10) gemäss einem der Ansprüche 1 bis 3, und
- mindestens einen Stab (1) oder Stift (30), der sich zwischen den besagten Stabklemmen (10) erstreckt und festzuhalten ist, wobei der besagte Stab oder Stift aus einem nicht ferromagnetischen Materialkern (2) hergestellt ist, der durch eine nicht elektrisch leitende Hülle (3) umgeben ist.

5. Äusseres Befestigungssystem nach Anspruch 4, bei dem der Kern (2) und die Hülle (3) eines Stabes (1) oder Stiftes (30) zusammengehalten werden bei Ausübung eines Momentes um die Hauptachse (5) des Stabes (1) oder Stiftes (30) in solch einer Weise, dass jegliche der besagten Klemmelemente (10) entlang dem Umkreis der Hülle (3) zu gleiten beginnen, bevor die Oberflächen zwischen dem Kern (2) und der Hülle (3) durch das anwachsende Moment getrennt werden.

6. Äusseres Befestigungssystem nach einem der Ansprüche 4 oder 5, bei dem der antimagnetische Kernkörper (2, 32) von jedem der besagten Klemmelemente (10) und jeglicher Stab (1) oder Stift (30) aus Titan, Aluminium oder Kohlefaser bestehen und/oder nichtleitende Hüllenteile (3, 33) aus Harzmaterial bestehen.

7. Äusseres Befestigungssystem nach Anspruch 6, bei dem der antimagnetische Kernkörper (2, 32) aus Kohlefaser besteht und der nichtleitende Hüllenteil (3, 33) aus co-extrudiertem Harzmaterial innerhalb eines Harzübertragungsgussverfahrens (RTM- Verfahren) besteht.

## Revendications

1. Pince (10) pour un système de fixateur externe, adaptée pour serrer une barre (1) ou une goupille (30), comprenant un corps principale (32) antimagnétique et une surface (33) non-conducteur formant une partie de gaine couvrant essentiellement les surfaces extérieures du corps principal (32), **caractérisé en ce que** la pince (10) comprend des mâchoires (40) ayant des dents (41) sur la surface de serrage prévues pour capturer une barre (1) ou une goupille (30), où la longueur de ces dents (41) est plus petite que l'épaisseur de la gaine (3) isolante de la dite barre (1) ou de la goupille (30).

2. Pince (10) selon la revendication 1, où le corps principal (32) antimagnétique est fabriqué en titane ou aluminium ou en fibre de carbone et/ou la partie de gaine (33) non-conducteur est fabriquée en matériau de résineux.

3. Pince (10) selon la revendication 2, où la partie principale (32) antimagnétique est fabriquée en fibre de carbone et la partie de gaine (33) non-conducteur est fabriquée dans un matériau résineux co-extrudé par un procédé de moulage de transfert de résine (procédé RTM).

4. Un système de fixateur externe IRM sûr, comprenant:
- au moins deux pinces de barre (10) selon l'une des revendications 1 à 3, et
- au moins une barre (1) ou goupille (30) pour être capturée par et s'étendant entre lesdites pinces de barre (10), où ladite barre ou ladite goupille sont fabriquées dans une partie principale en matériau antiferromagnétique (2) entourée par une gaine électriquement non-conducteur.

5. Système de fixateur externe selon la revendication 4, où la partie principale (2) et la gaine (3) de la barre (1) ou de la goupille (30) sont tenues ensembles à l'encontre de l'exercice d'un couple de rotation autour de l'axe principale (5) de la barre (1) ou de la goupille (30) d'une telle manière que chacune des dites pinces (10) va commencer à glisser le long de la circonférence de la gaine (3), avant que les surfaces du noyau (2) et da la gaine (3) sont séparées par un couple grandissant.

6. Système de fixateur externe selon une des revendications 4 ou 5, où le corps principal (2, 32) antimagnétique de chacune des pinces (10) et de chaque barre (1) ou goupille (30) est fabriqué en titane ou aluminium ou en fibre de carbone, et/où la partie de gaine non-conducteur (3, 33) est fabriquée en matériau résineux.

7. Système de fixateur externe selon la revendication 6, où la partie principale antimagnétique (2, 32) est fabriquée en fibre de carbone et la partie de gaine non-conducteur (3, 33) est fabriquée dans un matériau résineux co-extrudé, à l'aide d'un procédé de moulage de transfert de résine (procédé RTM).
